# FASCICULE DE BREVET EUROPEEN

(11) **EP 1 706 175 B1**
(45) Date de publication et mention de la délivrance du brevet: **31.08.2011**
(21) Numéro de dépôt: 04736641.4
(22) Date de dépôt: 11.06.2004
(51) Int. Cl.: A61N 1/04, A61N 1/00, A61N 1/39, A61N 1/18, A61N 1/32

(54) **DISPOSITIF D'ELECTROSTIMULATION MUSCULAIRE**
EINRICHTUNG ZUR ELEKTRISCHEN STIMULATION VON MUSKELN
MUSCULAR ELECTROSTIMULATION DEVICE

(30) Priorité: 30.12.2003 EP 03104992
(43) Date de publication de la demande: 04.10.2006
(60) Demande divisionnaire: 11150084.9
(73) Titulaire: Guillarme, Luc, 71100 Chalon sur Saone (FR)
(72) Inventeur: Guillarme, Luc, 71100 Chalon sur Saone (FR)
(74) Mandataire: Verriest, Philippe
(86) Numéro de dépôt international: PCT/EP2004/006358
(87) Numéro de publication internationale: WO 2005/068013

(56) Documents cités:
- WO-A-90/11796
- US-A- 5 109 842
- US-A- 5 562 717
- US-A- 5 800 501
- US-A1- 2002 188 332
- US-B1- 6 246 915

## Description

L'invention concerne un nouveau dispositif d'électrostimulation musculaire dans lequel l'alimentation électrique des électrodes est assurée par un électrostimulateur, en réponse à une onde sonore générée par l'utilisateur, lorsqu'il souffle dans un embout selon le dispositif revendiqué. Ce dispositif est particulièrement adapté dans le cadre de méthodes de musculation abdominale et de rééducation abdominale ou périnéale.

L'invention concerne également une électrode nouvelle, en forme d'ailes de papillon et comportant deux électrodes individuelles, accolées et séparées par un composant non-conducteur; une telle électrode permet une stimulation perfectionnée des muscles et par conséquent un effet thérapeutique plus important.

Il a été décrit pour la première fois, dans le document EP-B-0 519 646, une méthode de musculation associant le souffle et la contraction des muscles abdominaux.

Cette méthode consiste à provoquer l'expiration de l'air contenu dans les poumons en deux phases continues et successives, une première phase d'expiration thoracique sans prise d'air préalable, suivie d'une seconde phase d'expiration abdominale, consistant en une contraction isotonique des muscles abdominaux.

Cette méthode prévoit également le maintien en position semi-ouverte des deux machoires au moyen d'un embout, de telle sorte à favoriser l'exsufflation.

De manière avantageuse, elle préconise la mise en oeuvre d'une électro-neurostimulation abdominale ou périnéale concomitante, par délivrance d'impulsions électriques au niveau des muscles abdominaux ou de la musculature périnéale, générant un effet tétanisant trophique polyvalent tant sur l'abdomen pour une musculation abdominale que sur le périnée pour une rééducation périnéale.
Dans le but de corréler le souffle et le déclenchement de la stimulation électrique, ce document envisage également de relier l'embout d'exsufflation à félectroneuro-stimulateur au moyen d'un tube flexible.

Par la suite, le document FR 2 793 946 s'est attaché à corréler, de manière beaucoup plus précise, la délivrance des impulsions électriques par l'électro-neuro-stimulateur avec la phase d'exsufflation proprement dite. Ainsi, ce document divulgue un dispositif comprenant un embout permettant l'exsufflation et comprenant des moyens susceptibles de déclencher un signal électrique de commande de l'électrostimulateur, sous l'action du souffle provenant de l'exsufflation, propre à induire le déclenchement des impulsions de l'électro-neuro-stimulateur. En pratique, il a été envisagé d'utiliser un couple d' ailettes qui, en changeant de position sous la pression physique exercée par le souffle, ferme un circuit électrique générant les signaux de commande de l'électro-neuro-stimulateur. Document WO 90/11796 décrit un système selon le préambule de la revendication 1.

Dans une forme encore plus élaborée, il est envisagé de transmettre le signal électrique de commande par ondes radio ou équivalents, afin de s'affranchir de tout fils de raccordement entre le détecteur de souffle et l'électroneurostimulateur.

Si incontestablement, ce dispositif permet d'établir une synergie respiratoire et musculaire, il reste perfectible sur différents aspects.

En particulier, il repose entièrement sur la pression exercée par le souffle, à savoir un paramètre physique fluctuant et de qualité irrégulière. Par ailleurs, dans ce contexte, les fonctions cruciales de capteur et d' organe de commande se trouvent concentrées dans l'embout, et donc aux mains de l'utilisateur.

Enfin, ce système s' avère délicat et peu pratique :
- Il nécessite le positionnement, sur la partie buccale de l'embout, d'un autre embout protecteur muni d'un filtre de manière à retenir l'humidité existante dans l'air exsufflé de l'utilisateur. En effet, à long terme, l'eau est un facteur dégradant du système électro-nique chargé de capter et déclencher la commande de l' électrostimulateur;
- le câble de raccordement entre le capteur souffle et l'électrostimulateur est une gêne au bon déroulement de la méthode pratiquée dans les deux positions assise/couchée ;
- le poids du dispositif intra-buccal nécessite de la part de l'utilisateur son maintien manuel, en particulier en position assise.

L'invention vise à coupler le déclenchement de l'électrostimulateur à un signal de commande, certes lié au souffle, mais ne dépendant plus strictement de la qualité dudit souffle, grâce à la coopération de deux entités physiques séparés, à savoir un embout et un électrostimulateur selon l'invention.

Ainsi, l'invention concerne un dispositif d'électrostimulation musculaire comprenant:
- un embout d' exsufflation apte à générer un signal de commande en fonction du souffle de l'utilisateur,
- un électrostimulateur connecté à des électrodes et apte à générer des signaux électriques en fonction du signal de commande généré par l'embout d'exsufflation, dans lequel l'embout d' exsufflation est muni de moyens aptes à générer, sous l'action du souffle de l'utilisateur, une onde sonore dans un domaine de fréquences donné correspondant au signal de commande; et dans lequel l'électrostimulateur comprend un capteur d'ondes sonores sensible au même domaine de fréquences, et est apte à déclencher la genèse desdits signaux électriques en fonction du signal de commande reçu de l'embout.

Selon une caractéristique de l'invention, lorsque l'utilisateur souffle dans l'embout d'exsufflation, une onde sonore dans un domaine de fréquences donné est générée et reconnue comme le signal de commande par l'électrostimulateur. Cet embout fonctionne donc à la manière d'un sifflet. Ainsi, il présente la capacité de transformer une donnée variable, à savoir le souffle de l'utilisateur, dont l'intensité en particulier est susceptible de fluctuer, en une donnée constante, une fréquence de son, reconnue par l'électrostimulateur .

Dans son agencement, cet embout d'exsufflation se caractérise par une partie intra-buccale, dont la forme a été significativement améliorée par le Demandeur. En effet, pour que la méthode de musculation soit efficace, le flux expiratoire doit être optimisé et en particulier, la résistance dans les voies aériennes doit être atténuée afin de créer un flux laminaire, lent ou rapide.

Or, le demandeur a mis en évidence que la résistance dans les voies aériennes était essentiellement liée à la glotte et à la bouche. En conséquence, la forme de la partie intra-buccale de l'embout a été adaptée afin de respecter les caractéristiques physiologiques de l'endroit le plus étroit du conduit trachéo-buccal, à savoir la glotte. De ce fait, cette partie intra-buccale se présente sous la forme d'un cylindre de diamètre équivalent à celui de la glotte, estimé entre 7 et 8.5 mm, préférentiellement 7,5 mm (diamètre moyen chez la femme) et 8 mm (diamètre moyen chez les hommes).

Dans sa partie externe, un embout destiné au dispositif selon l'invention comporte des ouvertures, également appelées orifices de sortie, ayant pour but de créer une onde sonore. Ces ouvertures sont préférentiellement au nombre de deux. En effet, l'air exsufflé dans l'embout, divisé en deux, augmente, par le phénomène de sous pression selon le système venturi, sa vitesse et crée l'onde sonore par résonance selon la fréquence souhaitée.

La superficie d'ouverture est calculée pour faciliter une sortie régulée du flux expiratoire et obtenir une onde stable, c' est à dire une fréquence constante captée par le micro situé au niveau de l' électrostimulateur.
La filtration électronique fixée à 8000 Hz (+/- 3000 Hz) est considérée comme la fréquence optimale, évitant la perturbation par des influences extérieures susceptibles de déformer l'onde sonore. La qualité de la fréquence (en puissance et en volume) garantit le bon fonctionnement de la stimulation.

Préférentiellement, les deux ouvertures sont situées sur la face supérieure de la partie externe de l'embout, manipulé par l'utilisateur.

D'une manière avantageuse, les parties externe et intra-buccale de l'embout sont séparées par une collerette de sécurité du positionnement en bouche, destinée à venir s' appuyer sur la surface labiale des dents antérieures de l'utilisateur.

La fréquence du son émis par l'embout sous l'action du souffle de l'utilisateur est avantageusement unique et indépendante de l'intensité dudit souffle. Elle est générée pendant toute la durée du souffle de l'utilisateur et sert de signal de commande pendant cette durée.

L 'électrostimulateur du dispositif d'électrostimulation musculaire selon l'invention est parfaitement adapté à l'embout tel que décrit ci-dessus.

En particulier, il comprend un capteur sous la forme d'un micro électronique intégré au boîtier de l'appareil, chargé de capter la fréquence du son résultant du souffle exercé par l'utilisateur au niveau de l'embout. Ce système embout/électrostimulateur dispense donc de toute connexion physique entre ces deux entités.

En réponse à l'onde sonore perçue, l'électrostimulateur génère des signaux électriques destinés à des électrodes. Les signaux électriques émis le sont préférentiellement sous la forme d'un courant bipolaire symétrique, reconnu le plus fiable électrophysiologiquement. En effet, le caractère bipolaire garantit l'utilisation de la stimulation électrique sans intempérance avec par exemple l'ostéosynthèse (plaque, vis, prothèse,...) ou un stérilet.

Les différents paramètres au niveau de l'électrostimulateur sont réglables. Dans un premier temps, des valeurs dites "standard" telles que :
- une fréquence égale à 100 Hz,
- la largeur d'impulsion égale à 1 milliseconde,
- la rampe d'arrivée et de départ du courant égale à 0,8 secondes,
peuvent être appliquées et donc programmées au niveau de 1' électrostimulateur.

Dans la mesure où, dans cette méthode, les stimulations électriques ne font qu' assister la contraction musculaire volontaire de l'utilisateur et ou le souffle issu de la contraction abdominale garantit la bonne orientation des flux de pression intracorporels, les paramètres du courant importent peu. Cependant, il est possible de modifier ces paramètres pour des objectifs thérapeutiques ciblés.

La délivrance des signaux électriques aux électrodes est déclenchée par la capture au niveau du micro, situé dans l'électrostimulateur, de l'onde sonore émise par l'embout, à condition qu' elle appartienne au domaine de fréquences ou qu' elle corresponde à la fréquence unique, reconnus par ledit micro.

La délivrance de ces signaux électriques est maintenue aussi longtemps que le capteur perçoit cette onde sonore. D'une manière préférentielle, l'électrostimulateur est programmé de façon à ce que les signaux électriques perdurent quelques millisecondes après l'arrêt de l'émission de l'onde sonore au niveau de l'embout, et donc de sa capture au niveau de l'électrostimulateur. De fait, ceci invite l'utilisateur à prolonger le travail d'exsufflation et de contraction musculaire.
Par ailleurs, l' électrostimulateur est connecté aux électrodes grâce à des câbles électriques, préferentiellement doubles ou quadruples.

Dans le contexte propre à ce dispositif, le Demandeur a également optimisé la forme des électrodes applicables à la musculation abdominale et à la rééducation abdominale ou périnéale.

Alors que les électrodes couramment utilisées sont de forme ovale, ronde ou carrée, des électrodes en forme de boomerang, préférentiellement avec une angulation comprise entte 100 et 110°, s'avèrent particulièrement adaptées aux utilisations envisagées pour le dispositif revendiqué. De telles électrodes sont dénommées électrodes ABDO-MG®.

Le Demandeur a également montré que le positionnement desdites électrodes était particulièrement important pour l'efficacité des méthodes d'électrostimulation musculaire selon l'invention.

Ainsi, il a été déterminé que le point phrénique et la zone costoxiphoïdienne correspondaient aux zones d'insertion des muscles sollicités lors de la contraction abdominale corrélée au souffle. De ce fait, une première électrode est appliquée dans cette zone, située juste en dessous du sternum. Dans le cas d'une électrode en forme de boomerang, l'angulation comprise entre 100 et 110° est orientée vers le bas, mimant l'angulation costo-sternale.

Dans la musculation ou rééducation abdominale, cette électrode est couplée à une autre électrode située dans un axe vertical, au-dessus du pubis et dans une orientation opposée. Deux électrodes supplémentaires peuvent être ajoutées de manière à former un cercle concentrique.

Dans le cas de la musculation ou rééducation périnéale, l'utilisation des électrodes connues donne lieu à un certain nombre de problèmes. Généralement des sondes endocavitaires sont utilisées dont la mise en place est très désagréable pour l'utilisateur. De plus, chez certains peuples et dans certaines cultures, l'introduction de telles sondes n'est pas permise pour des raisons religieuses. La manipulation peu hygiénique de ces sondes peut également augmenter les chances d'une infection ou d'une inflammation tandis que le fait que toujours deux électrodes sont utilisée pose souvent des problèmes pratiques à l'utilisateur vu le nombre d'éléments à appliquer sur le corps avec les fils de connexion.

La présente invention propose de résoudre tous ces problèmes par l'utilisation pour la rééducation périnéale d'une électrode nouvelle, en forme d'ailes de papillon, comportant deux électrodes accollées et individuelles, reliées chacune par un câble distinct à l'électrostimulateur et séparées l'une de l'autre par un composant isolant. En positionnant cette nouvelle électrode sur le centre tendineux du périné, elle agit directement sur les muscles concernés et on obtient une stimulation améliorée et en même temps un effet thérapeutique plus important. A noter que l'utilisation de cette électrode n'est pas limitée à un dispositif d'électrostimulation musculaire tel que décrit ci-dessus mais que la nouvelle électrode selon l'invention peut également être utilisée dans d'autres applications et avec d'autres dispositifs.

Une seule électrode suffit à une rééducation proprioceptive et stimulante.

Avantageusement, une telle électrode est couplée à une ou plusieurs électrodes ABDO-MG®.

La manière dont l'invention peut être réalisée et les avantages qui en découlent ressortiront mieux des exemples de réalisation qui suivent, donnés à titre indicatif et non limitatif, à l'appui des figures annexées.
La figure 1 représente un prototype d'un électrostimulateur destiné au dispositif selon l'invention.
La figure 2 est une représentation schématique en perspective d'un embout conforme à l'invention.
La figure 3 est une représentation schématique en section longitudinale de la partie intra-buccale d'un embout conforme à l'invention.
La figure 4 est une représentation schématique d'une électrode abdominale.
La figure 5 illustre les positionnement des électrodes pour une rééducation abdominale à 2 électrodes (A) ou 4 électrodes (B).

Les figure 6A-6D donnent des représentations schématiques de différentes formes de réalisation de la nouvelle électrode périnéale.

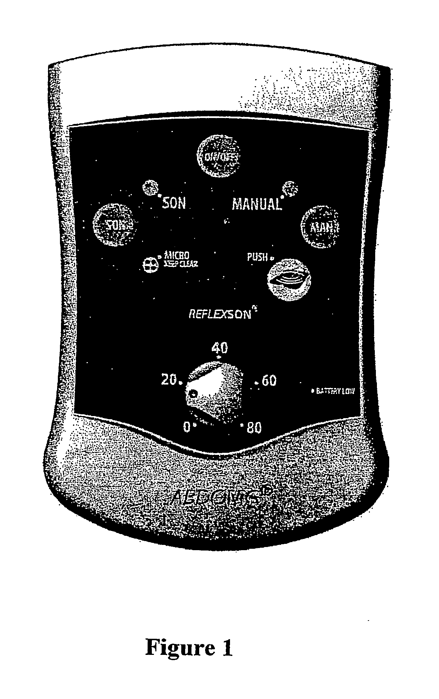

Un prototype d'un électrostimulateur selon l'invention est représenté à la figure 1. Il se présente sous la forme d'un boîtier de forme et de dimensions adaptées à une prise en mains par l'utilisateur, pour effectuer des réglages ou des commandes.

La partie destinée à la réception du signal se trouve dans la moitié supérieure du boîtier. Après avoir mis l'électrostimulateur sous tension via la commande ON/OFF, l'option "SON" est sélectionnée en appuyant sur le bouton correspondant. La détection de l'onde sonore se fait gràce à la présence d'un micro électronique intégré au boîtier, dont la surface est à protéger.

D'une manière alternative, cet électrostimulateur peut être utilisé en dehors du dispositif selon l'invention, par sélection du mode manuel. Dans ce mode, l'utilisateur doit appuyer sur le bouton "PUSH" pour générer les signaux électriques et ce, pendant toute la durée de délivrance souhaitée.

La partie inférieure du boîtier comprend un bouton de réglage de l'intensité des signaux électriques à délivrer. Le réglage de l'intensité s'effectue par l'utilisateur lui-même de sorte à atteindre un intensité efficace, agréable et confortable, compte-tenu qu' elle importe peu dans la qualité de la stimulation.

La partie postérieure de l'électrostimulateur, non visible sur la figure 1, comporte des fiches d'entrée pour la connexion des câbles, au nombre de deux ou quatre, permettant d'alimenter les électrodes. Par ailleurs, des boutons de réglage permettent de programmer ou de modifier différents paramètres relatifs à l'électrostimulateur (fréquence, largeur d'impulsion, rampe d'arrivée et de départ du courant...).

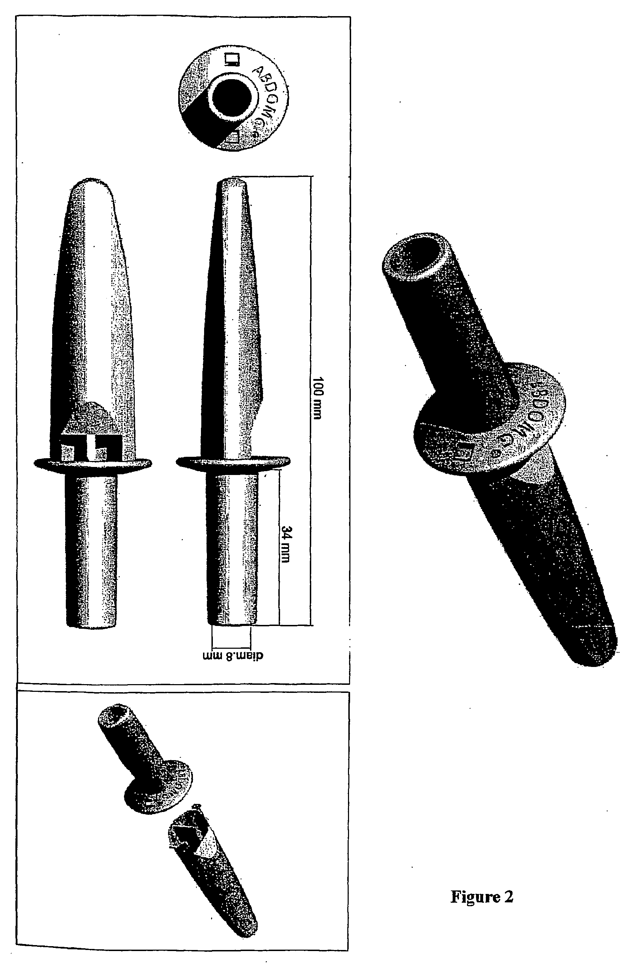

Un embout selon l'invention est représenté en perspective à la figure 2. Il est constitué de deux parties distinctes et dissociables :

La partie intra-buccale correspond à l'embout d'exsufflation, constitué d'un cylindre dont le diamètre interne optimisé est de 8 mm, et muni d'une saillie annulaire radiale destinée à venir d' appuyer sur la surface labiale des dents antérieures de l'utilisateur. La longueur optimisée du cylindre, de l'ordre de 34 mm, permet de concilier confort pour l'utilisateur et positionnement dans la bouche permettant la récupération du flux laminaire du souffle à la sortie de la glotte.

La collerette de sécurité du positionnement en bouche, située à l'opposé de l'extrémité destinée à pénétrer dans la bouche de l'utilisateur, comporte deux encoches en position horizontale et situées de part et d'autre du cylindre permettant l'insertion de la partie externe de l'embout par simple clipage.

La partie externe de l'embout, qui vient s'insérer au niveau de la collerette de l'embout d' exsufflation grâce à deux protubérances latérales de forme complémentaire, se présente comme un canal aplati dont l'extrémité à insérer dans la collerette est de dimensions légèrement supérieures au diamètre externe de l'embout d'exsufflation alors que l'autre extrémité est fermée.

Lorsque les deux parties sont associées, les seules ouvertures de la partie externe de l'embout sont constituées par les orifices de sortie, ayant la forme de deux carrés creusés, dont une arrête est définie par la collerette, et disposés de manière symétrique par rapport à l'axe vertical du canal. Ces orifices permettent la propagation de l'onde sonore générée à partir du souffle de l'utilisateur.

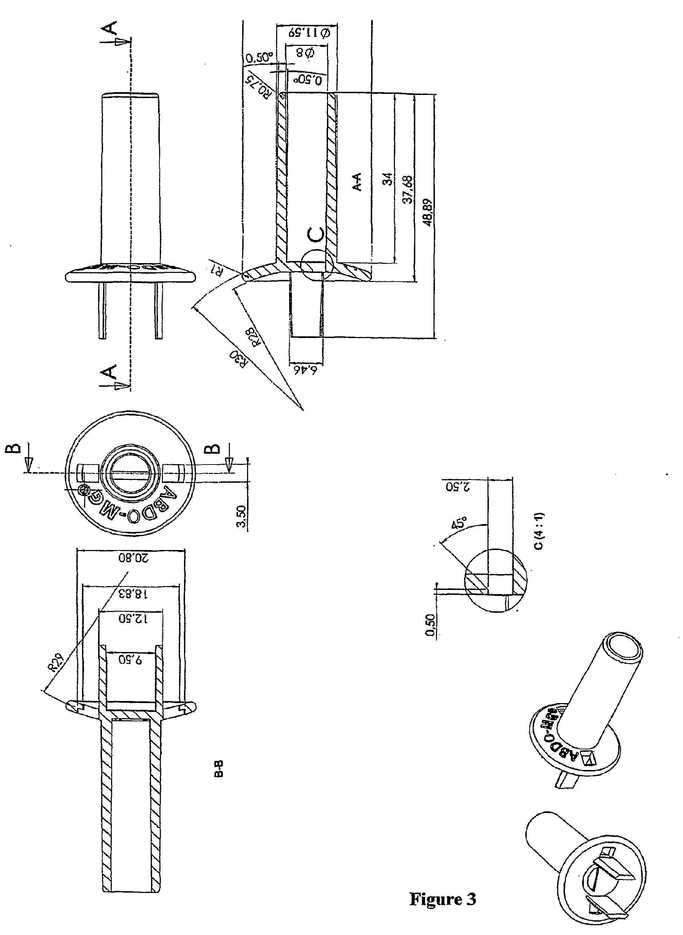

L'agencement de la partie intra-buccale munie de la collerette de l'embout selon l'invention est davantage détaillée dans les différentes vues de la figure 3: la vue B-B représente une section longitudinale dans un plan passant par les encoches de la collerette; la vue A-A représente également une section longitudinale mais dans un plan perpendiculaire; la vue C représente le détail de la zone de jonction entre le cylindre et la collerette. Il est à noter que l'embout d'exsufflation possède en outre au niveau de la collerette, entre les encoches et le diamètre externe du cylindre, deux arrêtes symétriques, destinées à être introduites dans le canal de la partie extérieure de l'embout pour consolider l'association des deux parties de embout.

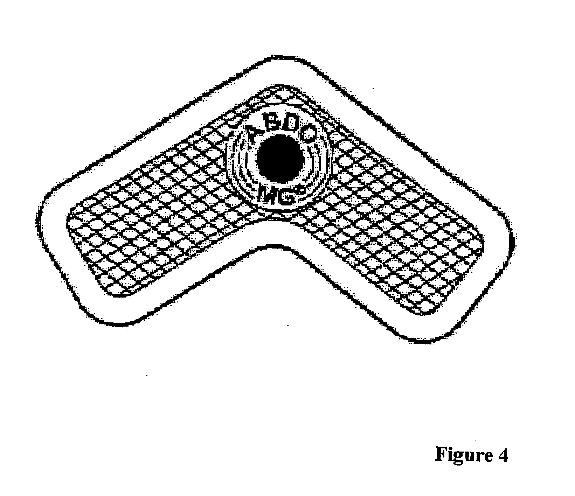

La figure 4 illustre une électrode en forme de boomerang selon l'invention. La forme, les dimensions, et en particulier l'angulation de 110°, permettent de superposer cette électrodes à la zone d'insertion des muscles impliqués dans la musculation abdominale basée sur le souffle.

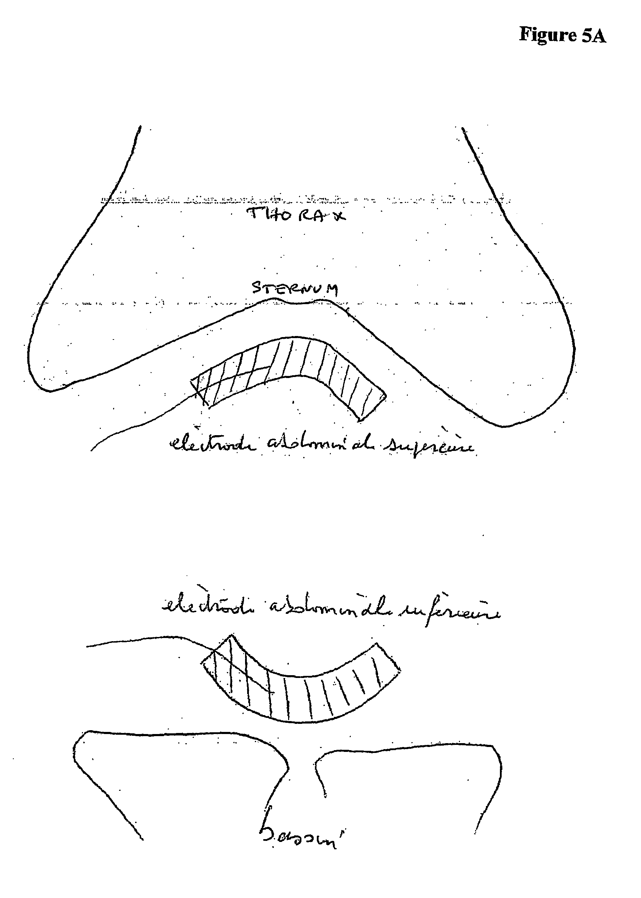

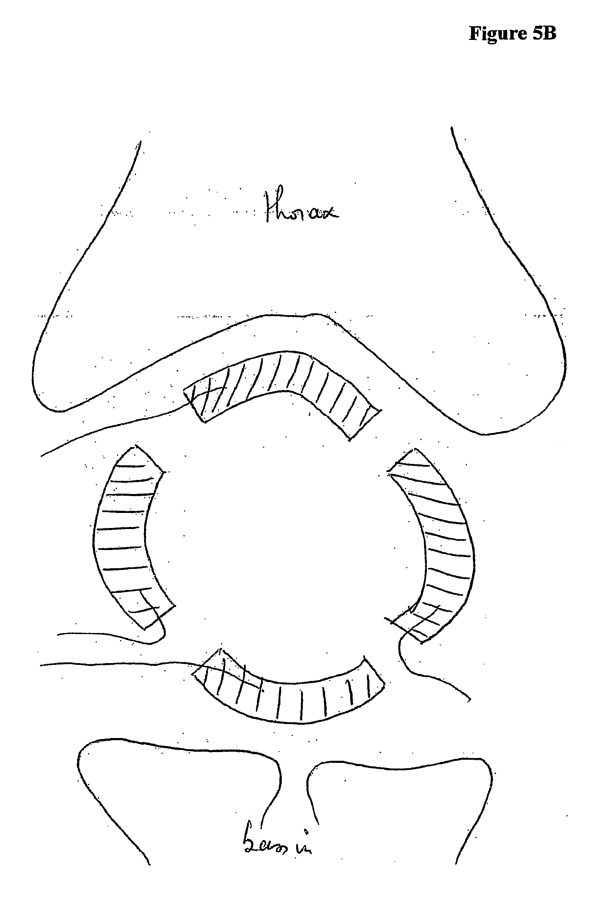

Dans le cadre de la rééducation abdominale, deux (Figure 5A) ou quatre (Figure 5B) de ces électrodes sont positionnées comme indiquées.

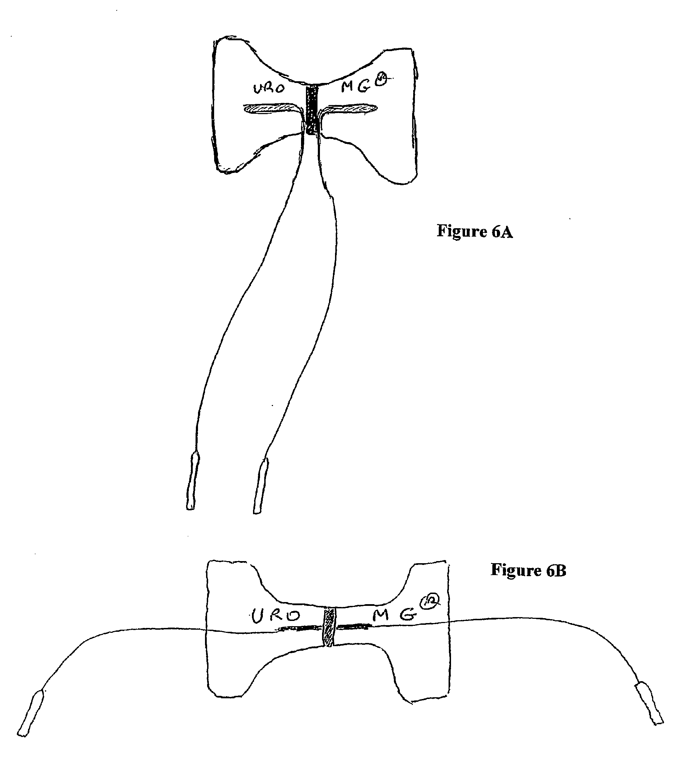

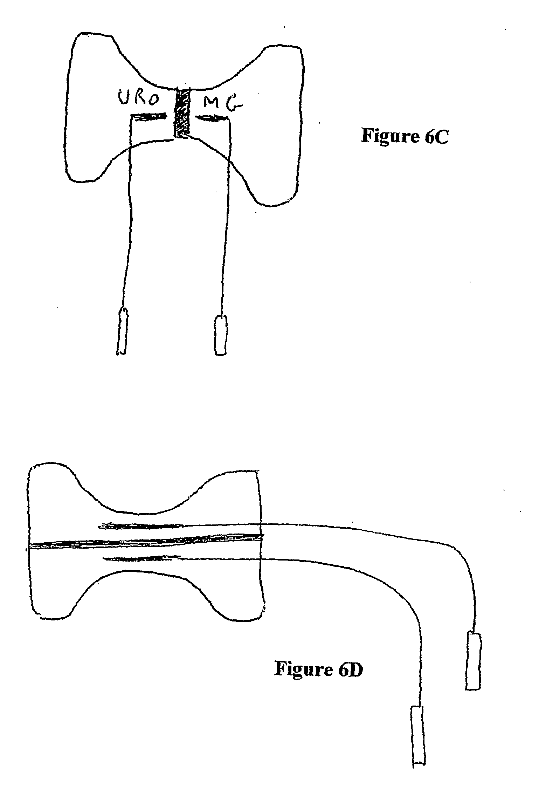

La figure 6 illustre une électrode périnéale selon l'invention, en forme d'ailes de papillon, comportant deux électrodes individuelles accolées, séparées l'une de l'autre par un composant non conducteur et reliées individuellement à l'électrostimulateur. Les deux électrodes et les deux câbles électriques qui les relient au générateur peuvent être agencés de manière différente.

Selon les Figures 6A et 6C, chaque électrode individuelle est constituée par une aile du papillon de façon à ce que l'électrode périnéale présente un axe de symmétrie transversal, les fils de connexion étant placés sur l'électrode dans une direction essentiellement parallèle à cet axe de symmétrie transversal.

Dans le mode d'exécution selon la Figure 6B, la disposition des électrodes individuelles est la même comme dans la Figure 6A, les fils de connexion étant toutefois placés essentiellement parallèlement à un axe de symmétrie longitudinal.

Dans le mode d'exécution selon la Figure 6D, l'électrode périnéale présente un axe de symmétrie longitudinal et une première électrode individuelles est située d'un côté de cet axe de symmétrie longitudinal, l'autre électrode individuelle étant située de l'autre côté de cet axe.

Dans le cadre de la rééducation périnéale, l'électrode spécifique périnéale est positionnée sur le centre tendineux du périnée. Des électrodes abdominales selon l'invention peuvent être couplées à cette électrode périnéale spécifique pour réaliser la pratique abdomino-périnéale expiratoire.

Il ressort clairement de la description que le dispositif revendiqué associant l'embout, l'électrostimulateur et les électrodes, et donc de fait destiné à des méthodes de musculation, est inventif. Il est cependant à noter que chaque entité à part entière est inventive et peut donc être utilisée séparément, dans un autre contexte : par exemple, les électrodes peuvent être alimentées par un générateur quelconque; l'électrostimulateur peut être actionné manuellement et donc dissocié de l'embout ; l'embout peut servir à déclencher tout autre appareil électrique pourvu d 'un capteur d' onde sonore adéquat.

## Revendications

1. Système d'électrodes périnéales en forme d'ailes de papillon **caractérisé en ce que** ledit système d'électrodes comporte deux électrodes individuelles accolées, séparées l'une de l'autre par un composant non conducteur.

2. Système d'électrodes selon la revendication 1 dans lequel chaque électrode individuelle est reliée à un électrostimulateur.

3. Système d'électrodes selon la revendication 1 ou 2 dans lequel chaque électrode individuelle a essentiellement la forme d'une aile de papillon et dans lequel les deux électrodes individuelles sont disposées de façon symétrique par rapport à l'axe de symétrie transversal du système d'électrodes périnéales

4. Système d'électrodes selon la revendication 1 ou 2 dans lequel chaque électrode individuelle s'étend sur une partie des deux ailes du papillon et dans lequel les deux électrodes individuelles sont disposées de façon symétrique par rapport à l'axe de symétrie longitudinal du système d'électrodes périnéales .

## Claims

1. A system of perineal electrodes with the shape of butterfly wings **characterized in that** said system of electrodes includes two individual electrodes placed side by side, separated from each other by a non-conductive component.

2. The system of electrodes according to claim 1, wherein each individual electrode is connected to an electrostimulator.

3. The system of electrodes according to claim 1 or 2, wherein each individual electrode essentially has the shape of a butterfly wing and wherein both individual electrodes are positioned symmetrically with respect to the transverse axis of symmetry of the system of perineal electrodes.

4. The system of electrodes according to claim 1 or 2, wherein each individual electrode extends over a portion of both wings of the butterfly and wherein both individual electrodes are positioned symmetrically with respect to the longitudinal axis of symmetry of the system of perineal electrodes.

## Patentansprüche

1. Perinealelektrodensystem in Schmetterlingsflügelform, **dadurch gekennzeichnet, dass** das Elektrodensystem zwei einzelne verbundene Elektroden aufweist, die voneinander durch ein nicht leitendes Bauteil getrennt sind.

2. Elektrodensystem nach Anspruch 1, wobei jede einzelne Elektrode mit einem Elektrostimulator verbunden ist.

3. Elektrodensystem nach Anspruch 1 oder 2, wobei jede einzelne Elektrode im Wesentlichen die Form eines Schmetterlingsflügels hat und wobei die zwei einzelnen Elektroden im Verhältnis zur transversalen Symmetrieachse des Perinealelektrodensystems symmetrisch angeordnet sinn.

4. Elektrodensystem nach Anspruch 1 oder 2, wobei sich jede einzelne Elektrode über einen Abschnitt der zwei Schmetterlingsflügel erstreckt und wobei die zwei einzelnen Elektroden im Verhältnis zur länglichen Symmetrieachse des Perinealelektrodensystems symmetrisch angeordnet sind.
